# EUROPEAN PATENT APPLICATION

(11) **EP 4 268 746 A1**
(43) Date of publication of application: **01.11.2023**
(21) Application number: 23170230.9
(22) Date of filing: 27.04.2023
(51) Int. Cl.: A61B 18/14, A61B 5/00, A61B 18/00

(54) **BASKET CATHETER WITH FORCE SENSOR HAVING BAYONET MOUNT**

(30) Priority: 28.04.2022 US 202263336094 P; 28.04.2022 US 202263336023 P; 15.11.2022 US 202218055481; 29.03.2023 US 202318192332
(71) Applicant: BIOSENSE WEBSTER (ISRAEL) LTD., 2066717 Yokneam (IL)
(72) Inventor: BEECKLER, Christopher Thomas, Irvine, 92618 (US); PAPAIOANNOU, Athanassios, Irvine, 92618 (US); KEYES, Joseph Thomas, Irvine, 92618 (US); OKARSKI, Kevin Mark, Irvine, 92618 (US); LICHTER, Justin George, Irvine, 92618 (US)
(74) Representative: Carpmaels & Ransford LLP

(57) **Abstract**

The disclosed technology includes a medical probe including a tubular shaft, a contact force sensor assembly, a spine retention hub, and an expandable basket assembly. The contact force sensor can include a first bayonet mount portion and the spine retention hub can include a second bayonet mount portion to couple the spine retention hub to the contact force sensor by interlocking with the first bayonet mount portion. The expandable basket assembly can include a plurality of spines and at least one electrode coupled to each of the plurality of spines. Each of the plurality of spines can be configured to bow radially outward from the longitudinal axis when the expandable basket assembly is transitioned from a collapsed form to an expanded form.

## Description

### REFERENCE TO RELATED APPLICATIONS

This application claims the benefit of priority under 35 U.S.C. § 119 to prior filed U.S. Provisional Patent Application No. 63/336,094 (Attorney Docket No. BI06693USPSP1) filed on April 28, 2022, prior filed U.S. Provisional Patent Application No. 63/336,023 (Attorney Docket No. BIO6675USPSP1) filed on April 28, 2022 and is a continuation-in-part application under 35 U.S.C. § 120 to U.S. Patent Application No. 18/055,481 (Attorney Docket No. BI06693USNP1) filed on November 15, 2022, the entire contents of each of which is hereby incorporated by reference as if set forth in full herein.

### FIELD

The present invention relates generally to medical devices, and in particular catheters with electrodes, and further relates to, but not exclusively, catheters suitable for use to induce irreversible electroporation (IRE) of cardiac tissues.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art tend to utilize radiofrequency (RF) electrical energy to heat tissue. RF ablation can have certain rare drawbacks due to operator's skill, such as heightened risk of thermal cell injury which can lead to tissue charring, burning, steam pop, phrenic nerve palsy, pulmonary vein stenosis, and esophageal fistula. Cryoablation is an alternative approach to RF ablation that can reduce some thermal risks associated with RF ablation but may present tissue damage due to the very low temperature nature of such devices. Maneuvering cryoablation devices and selectively applying cryoablation, however, is generally more challenging compared to RF ablation; therefore cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode catheters was previously proposed in the patent literature. Examples of systems and devices configured for IRE ablation are disclosed in U.S. Patent Pub. Nos. 2021/0161592A1, 2021/0169550A1, 2021/0169567A1, 2021/0169568A1, 2021/0177503A1, 2021/0186604A1, and 2021/0196372A1, each of which are incorporated herein by reference and attached in the appendix to priority application U. S. Provisional Patent Application No. 63/336,094.

Regions of cardiac tissue can be mapped by a catheter to identify the abnormal electrical signals. The same or different catheter can be used to perform ablation. Some example catheters include a number of spines with electrodes positioned thereon. The electrodes are generally attached to the spines and secured in place by soldering, welding, or using an adhesive. Furthermore, multiple linear spines are generally assembled together by attaching both ends of the linear spines to a tubular shaft (e.g., a pusher tube) to form a spherical basket. Due to the small size of the spines and the electrodes, however, adhering the electrodes to the spines and then forming a spherical basket from the multiple linear spines can be a difficult task, increasing the manufacturing time and cost and the chances that the electrode fails due to an improper bond or misalignment. What is needed, therefore, are devices and methods of forming an improved basket assembly that can help to reduce the time required for manufacturing the basket assembly, alternative catheter geometries, and alternative electrode shapes and sizes in general.

### SUMMARY

The disclosed technology can include a medical probe having a tubular shaft, a contact force sensor assembly, a spine retention hub, and an expandable basket assembly. The tubular shaft can include a proximal end and a distal end and extending along a longitudinal axis of the medical probe. The contact force sensor assembly can be disposed at the distal end of the tubular shaft and be configured to detect a force applied to the medical probe. The contact force sensor assembly can include a first bayonet mount portion.

The spine retention hub can be coupled to the contact force sensor assembly. The spine retention hub can include a second bayonet mount portion that can be configured to couple the spine retention hub to the contact force sensor assembly by interlocking with the first bayonet mount portion.

The expandable basket assembly can be coupled to the spine retention hub. The expandable basket assembly can include a plurality of spines and at least one electrode coupled to each of the plurality of spines. Each of the plurality of spines can extend along the longitudinal axis and be configured to bow radially outward from the longitudinal axis when the expandable basket assembly is transitioned from a collapsed form to an expanded form.

The first bayonet mount can include a slot formed into the contact force sensor and the second bayonet mount can include a protrusion extending from the spine retention hub. The slot can be configured to receive the protrusion. The slot and the protrusion can each be generally L-shaped. The slot can include a first slot portion that can extend generally longitudinally into the contact force sensor assembly from a distal end of the contact force sensor assembly and a second slot portion that can extend generally transversely from an end of the first slot portion. The protrusion can include a first protrusion portion extending generally longitudinally away from the spine retention hub and a second protrusion portion extending generally transversely from an end of the first protrusion portion.

The first bayonet mount can include at least two slots formed into the contact force sensor and the second bayonet mount can include at least two protrusions extending from the spine retention hub. The at least two slots can be configured to receive the at least two protrusions.

The first bayonet mount can include a protrusion extending from the contact force sensor assembly and the second bayonet mount can include a slot formed into the spine retention hub. The slot can be configured to receive the protrusion. The slot can have a generally L-shaped recess and the protrusion can be a generally L-shaped member.

The protrusion can include a first protrusion portion extending generally longitudinally from the contact force sensor assembly and a second protrusion portion extending generally transversely from an end of the first protrusion portion. The slot can be a first slot portion extending generally longitudinally from a proximal end of the spine retention hub and a second slot portion extending generally transversely from an end of the first slot portion. The first bayonet mount can include at least two protrusions extending from the contact force sensor and the second bayonet mount can include at least two slots formed into the spine retention hub. The at least two slots can be configured to receive the at least two protrusions.

The contact force sensor can include a body having a generally cylindrical shape, a coil configured to generate a magnetic field, a sensor configured to detect the magnetic field generated by the coil, and a deflection portion configured to permit the body to deflect when a force is applied to the contact force sensor. The deflection portion can include a helical spring. The helical spring can be formed into the body of the contact force sensor by forming a helical cut into the body of the contact force sensor.

The coil can be disposed at a proximal end of the contact force sensor assembly and the sensor can be disposed at a distal end of the contact force sensor assembly.

The spine retention hub can include a spray port configured to direct a fluid toward the at least one electrode.

Each of the plurality of spines can include at least one retention member extending generally transverse to the spine. The at least one electrode can include a body defining a hollow portion extending through the body of the electrode. The body can be configured to receive each of the plurality of spines. The electrode can be retained by the at least one retention member. The at least one retention member can include a bow-shaped member. In other examples, the at least one retention member can include two bow-shaped members disposed in opposite direction and transverse to a longer length of each spine.

The at least one electrode can include a first electrode and a second electrode. The at least one retention member can include first and second sets of retention members spaced apart along each of the plurality of spines. The first set can include two bow-shaped members disposed in opposite direction and transverse to a longer length of each spine and the second set includes two bow-shaped members disposed in opposite direction and transverse to a longer length of each spine so that the first electrode can be captured between the first set of retention members and the second electrode can be captured between the second set of retention members.

The medical probe can include an electrically insulative jacket disposed between each of the plurality of spines and the at least one electrode, thereby electrically isolating the at least one electrode from each of the plurality of spines. The medical probe can include a wire disposed inside the insulative jacket. The wire can be electrically connected to the at least one electrode.

Each of the plurality of spines can include a material selected from a group consisting of nitinol, cobalt chromium, stainless steel, titanium, and combinations hereof. Each of the plurality of spines can be configured to form an approximately spherically-shaped or oblate-spheroid shaped basket assembly when in the expanded form.

The at least one electrode can include of a material selected from stainless steel, cobalt chromium, gold, platinum, palladium, and alloys hereof. The at least one electrode can be configured to deliver electrical pulses for irreversible electroporation. The pulses can include a peak voltage of at least 900 volts (V).

Additional features, functionalities, and applications of the disclosed technology are discussed herein in more detail.

### BRIEF DESCRIPTION OF THE DRAWINGS

FIG. 1 is a schematic pictorial illustration of a medical system including a medical probe whose distal end includes a basket assembly with electrodes, in accordance with an example of the disclosed technology;
FIG. 2 is a perspective view of a medical probe in an expanded form, in accordance with an example of the disclosed technology;
FIG. 3A illustrates the medical probe of Fig. 2 with only the underlying spine structure and one electrode disposed on one spine in accordance with an example of the disclosed technology;
FIG. 3B illustrates the spine structure as formed from a tube stock in accordance with an example of the disclosed technology;
FIG. 4 illustrates an exploded view of the medical probe of FIG. 2, in accordance with an example of the disclosed technology;
FIG. 5A illustrates a perspective view of a contact force sensor assembly and a spine retention hub of the medical probe, in accordance with an example of the disclosed technology;
FIGs. 5B and 5C illustrate exploded views of a contact force sensor assembly and a spine retention hub of the medical probe, in accordance with an example of the disclosed technology;
FIG. 6A illustrates a section view of a contact force sensor assembly and a spine retention hub of the medical probe, in accordance with an example of the disclosed technology;
FIG. 6B illustrates and exploded section view of a contact force sensor assembly and a spine retention hub of the medical probe, in accordance with an example of the disclosed technology;
FIGs. 7A-7C illustrate section and detail views of a contact force sensor assembly and a spine retention hub of the medical probe to illustrate how the contact force sensor assembly and spine retention hub are coupled together, in accordance with an example of the disclosed technology;
FIG. 8A is a schematic pictorial illustration showing a perspective view of another example medical probe in an expanded form, in accordance with another example of the disclosed technology; and
FIG. 8B is a schematic pictorial illustration showing a perspective view of the medical probe of 8A showing the spines, in accordance with the disclosed technology.

### DETAILED DESCRIPTION

The following detailed description should be read with reference to the drawings, in which like elements in different drawings are identically numbered. The drawings, which are not necessarily to scale, depict selected examples and are not intended to limit the scope of the invention. The detailed description illustrates by way of example, not by way of limitation, the principles of the invention. This description will clearly enable one skilled in the art to make and use the invention, and describes several examples, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 71% to 110%.

As used herein, the terms "patient," "host," "user," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example. As well, the term "proximal" indicates a location closer to the operator or physician whereas "distal" indicates a location further away to the operator or physician.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" (sometimes referred to as "monopolar") refers to ablation scheme utilizing a current path between two electrodes where one electrode including a high current density and high electric flux density is positioned at a treatment site, and a second electrode including comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal including a positive-voltage phase pulse (referred to herein as "positive phase") and a negative-voltage phase pulse (referred to herein as "negative phase"). "Monophasic pulse" refers to an electrical signal including only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape including an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The term "temperature rating", as used herein, is defined as the maximum continuous temperature that a component can withstand during its lifetime without causing thermal damage, such as melting or thermal degradation (e.g., charring and crumbling) of the component.

The present disclosure is related to systems, methods or uses and devices which utilize end effectors including electrodes affixed to spines. Example systems, methods, and devices of the present disclosure may be particularly suited for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation.

RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

IRE as discussed in this disclosure is a non-thermal cell death technology that can be used for ablation of atrial arrhythmias. To ablate using IRE/PEF, biphasic voltage pulses are applied to disrupt cellular structures of the myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by programmed cell death or apoptosis, which is believed to leave less scar tissue as compared to other ablation modalities. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and methods for applying electrical signals from catheter electrodes positioned in the vicinity of myocardial tissue, preferably by applying a pulsed electric field effective to induce electroporation in the myocardial tissue. The systems and methods can be effective to ablate targeted tissue by inducing irreversible electroporation. In some examples, the systems and methods can be effective to induce reversible electroporation as part of a diagnostic procedure. Reversible electroporation occurs when the voltage applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210, the entirety of which is incorporated herein by reference and attached in the appendix to priority application U.S. Provisional Patent Application No. 63/336,094.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0161592A1, 2021/0169550A1, 2021/0169567A1, 2021/0169568A1, 2021/0177503A1, 2021/0186604A1, and 2021/0196372A1, the entireties of each of which are incorporated herein by reference and attached in the appendix to priority application U.S. Provisional Patent Application No. 63/336,094.

To deliver pulsed field ablation (PFA) in an IRE (irreversible electroporation) procedure, electrodes should contact the tissue being ablated with a sufficiently large surface area. As described hereinbelow, the medical probe includes a tubular shaft including proximal and distal ends, and a basket assembly at the distal end of the tubular shaft. The basket assembly includes a single unitary structure. The unitary structure can include a plurality of linear spines formed from a planar sheet of material and one or more electrodes coupled to each of the spines. The plurality of linear spines can converge at a central spine intersection including one or more cutouts. The cutouts can allow for bending of each spine such that the spines form an approximately spherical or oblate-spheroid basket assembly. It is noted that the cutouts (in various configurations described and illustrated in the specification) allows the basket to be compressed into a much smaller form factor when undeployed (or undergoing a retraction into a delivery sheath) without buckling or plastic deformation.

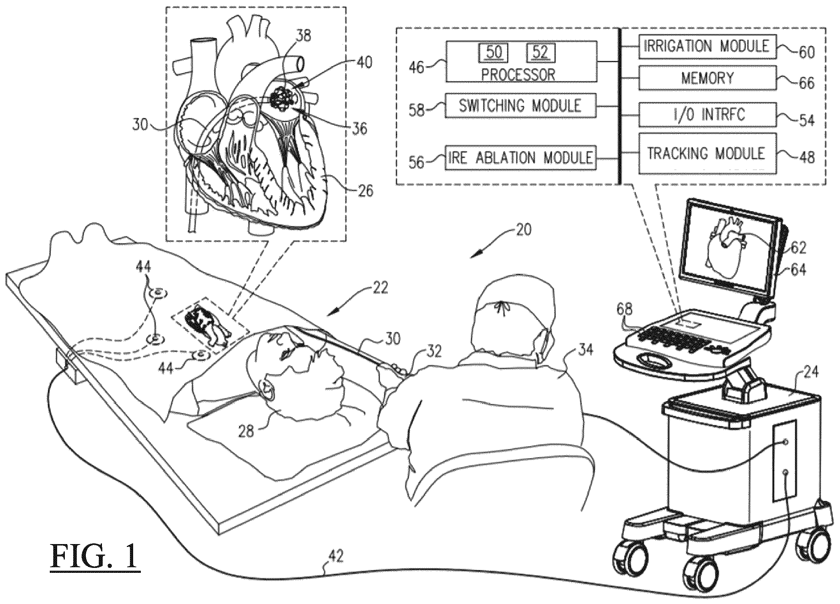

FIG. 1 is a schematic, pictorial illustration of a medical system 20 including a medical probe 22 and a control console 24, in accordance with an example of the present invention. Medical system 20 may be based, for example, on the CARTOR system, produced by Biosense Webster Inc. of 31 Technology Drive, Suite 200, Irvine, CA 92618 USA. In examples described hereinbelow, medical probe 22 can be used for diagnostic or therapeutic treatment, such as for performing ablation procedures in a heart 26 of a patient 28. Alternatively, medical probe 22 may be used, mutatis mutandis, for other therapeutic and/or diagnostic purposes in the heart or in other body organs.

Medical probe 22 includes a flexible insertion tube 30 and a handle 32 coupled to a proximal end of the tubular shaft. During a medical procedure, a medical professional 34 can insert probe 22 through the vascular system of patient 28 so that a distal end 36 of the medical probe 22 enters a body cavity such as a chamber of heart 26. Upon distal end 36 entering the chamber of heart 26, medical professional 34 can deploy a basket assembly 38 near the distal end 36 of the medical probe 22. Basket assembly 38 can include a plurality of electrodes 40 affixed to a plurality of spines 214. To start performing a medical procedure such as irreversible electroporation (IRE) ablation, medical professional 34 can manipulate a handle (either handle 32 or a separate handle, or both) to position distal end 36 so that electrodes 40 engage cardiac tissue at a desired location or locations. Upon positioning the distal end 36 so that electrodes 40 (disposed on the basket assembly 38) engage cardiac tissue, the medical professional 34 can activate the medical probe 22 such that electrical pulses are delivered by the electrodes 40 to perform the IRE ablation.

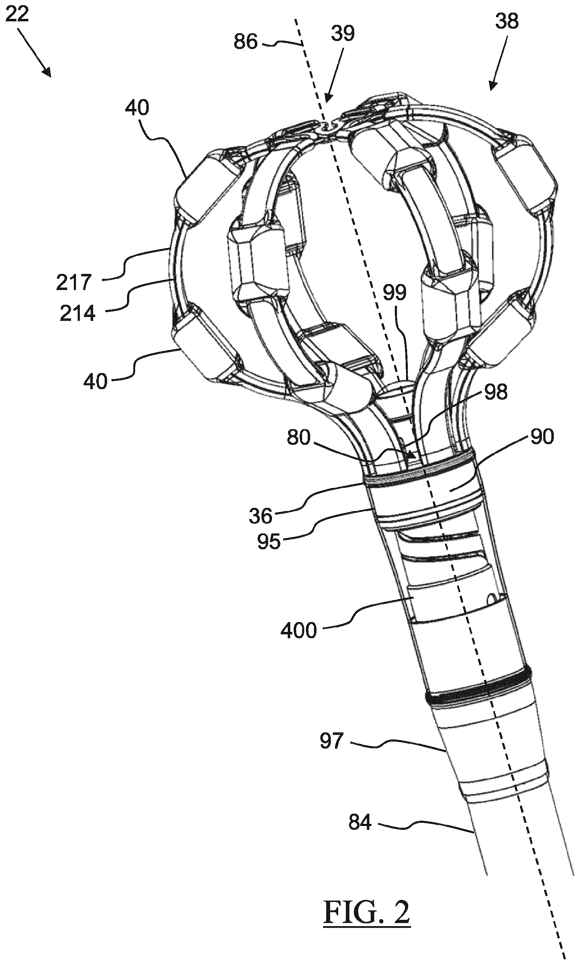

The medical probe 22 can include a guide sheath and a therapeutic catheter, wherein the guide sheath includes the flexible insertion tube 30 and the handle 32 and the therapeutic catheter includes the basket assembly 38, electrodes 40, a tubular shaft 84 (see FIGs. 2 through 4). The therapeutic catheter is translated through the guide sheath so that the basket assembly 38 is positioned in the heart 26. The distal end 36 of the medical probe 22 corresponds to a distal end of the guide sheath when the basket assembly 38 is contained within the flexible insertion tube 30, and the distal end 36 (of the tube 30) corresponds to a proximal portion (Fig. 2) of the basket assembly 38 when the basket assembly 38 is extended from the distal end of the guide sheath. The medical probe 22 can be alternatively configured to include a second handle on the therapeutic catheter and other features as understood by a person skilled in the pertinent art.

In the configuration shown in FIG. 1, control console 24 is connected, by a cable 42, to body surface electrodes, which typically include adhesive skin patches 44 that are affixed to patient 28. Control console 24 includes a processor 46 that, in conjunction with a tracking module 48, determines location coordinates of distal end 36 inside heart 26. Location coordinates can be determined based on electromagnetic position sensor output signals provided from the distal portion of the catheter when in the presence of a generated magnetic field. Location coordinates can additionally, or alternatively be based on impedances and/or currents measured between adhesive skin patches 44 and electrodes 40 that are affixed to basket assembly 38. In addition to being used for recording ECG signals or acting as location sensors during a medical procedure, electrodes 40 may perform other tasks such as ablating tissue in the heart.

As described hereinabove, in conjunction with tracking module 48, processor 46 may determine location coordinates of distal end 36 for tube 30 inside heart 26 based on impedances and/or currents measured between adhesive skin patches 44 and electrodes 40. Such a determination is typically after a calibration process relating the impedances or currents to known locations of the distal end has been performed. While examples presented herein describe electrodes 40 that are preferably configured to deliver IRE ablation energy to tissue in heart 26, configuring electrodes 40 to deliver any other type of ablation energy to tissue in any body cavity is considered to be within the spirit and scope of the present invention. Furthermore, although described in the context of being electrodes 40 that are configured to deliver IRE ablation energy to tissue in the heart 26, one skilled in the art will appreciate that the disclosed technology can be applicable to electrodes used for mapping and/or determining various characteristics of an organ or other part of the patient's 28 body.

Processor 46 may include real-time noise reduction circuitry 50 typically configured as a field programmable gate array (FPGA), followed by an analog-to-digital (A/D) signal conversion integrated circuit 52. The processor can be programmed to perform one or more algorithms and uses circuitry 50 and circuit 52 as well as features of modules to enable the medical professional 34 to perform the IRE ablation procedure.

Control console 24 also includes an input/output (I/O) communications interface 54 that enables control console 24 to transfer signals from, and/or transfer signals to electrodes 40 and adhesive skin patches 44. In the configuration shown in FIG. 1, control console 24 additionally includes an IRE ablation module 56 and a switching module 58.

IRE ablation module 56 is configured to generate IRE pulses including peak power in the range of tens of kilowatts. In some examples, the electrodes 40 are configured to deliver electrical pulses including a peak voltage of at least 900 volts (V). The medical system 20 performs IRE ablation by delivering IRE pulses to electrodes 40. Preferably, the medical system 20 delivers biphasic pulses between electrodes 40 on the spine. Additionally, or alternatively, the medical system 20 delivers monophasic pulses between at least one of the electrodes 40 and a skin patch.

In order to prevent blood coagulation, system 20 supplies irrigation fluid (e.g., a normal saline solution) to distal end 36 of tube 30 and to the proximal area of basket assembly 38. It is noted that irrigation fluid can be supplied through the flexible insertion tube 30. Control console 24 includes an irrigation module 60 to monitor and control irrigation parameters, such as the pressure and the temperature of the irrigation fluid. It is noted that while the preference for the exemplary embodiments of the medical probe is for IRE or PFA, it is within the scope of the present invention to also use the medical probe separately only for RF ablation (unipolar mode with an external grounding electrode or bipolar mode) or in combination with IRE and RF ablations sequentially (certain electrodes in IRE mode and other electrodes in RF mode) or simultaneously (groups of electrodes in IRE mode and other electrodes in RF mode).

Based on signals received from electrodes 40 and/or adhesive skin patches 44, processor 46 can generate an electroanatomical map 62 that shows the location of distal end 36 in the patient's body. During the procedure, processor 46 can present map 62 to medical professional 34 on a display 64, and store data representing the electroanatomical map in a memory 66. Memory 66 may include any suitable volatile and/or non-volatile memory, such as random-access memory or a hard disk drive.

In some examples, medical professional 34 can manipulate map 62 using one or more input devices 68. In alternative examples, display 64 may include a touchscreen that can be configured to accept inputs from medical professional 34, in addition to presenting map 62.

FIG. 2 is an illustration of a prototype showing a perspective view of a medical probe 22 including a basket assembly 38 in an expanded form when unconstrained, such as by being advanced out of an insertion tube lumen 80 at a distal end 36 of an insertion tube 30. Probe 22 may include a contact force sensor assembly 400 to determine contact force of the spines against cardiac tissues. It should be noted that the medical probe 22 illustrated in FIG. 2 lacks the guide sheath illustrated in FIG. 1. In the expanded form FIG. 2, spines 214 bow radially outwardly and in the collapsed form not shown the spines are arranged generally along a longitudinal axis 86 of insertion tube 30. For simplicity, only a single spine 214 has been marked with a reference number, but one of skill in the art will appreciate that the basket assembly 38 can include one or more spines 214 as illustrated in Fig. 2. Similarly only electrodes 40 and an insulative sleeve 217 on the single spine 214 are marked with references numbers, but one of skill in the art will appreciate that the basket assembly 38 can include one or more electrodes 40 and one or more insulative sleeves depending on the particular configuration. A plurality of electrically insulative sleeves 217 can be provided so that each jacket can be disposed between a respective spine 214 of the plurality of spines 214 and a respective electrode 40 of the plurality of electrodes 40, thereby electrically isolating the plurality of electrodes 40 from the plurality of spines 214.

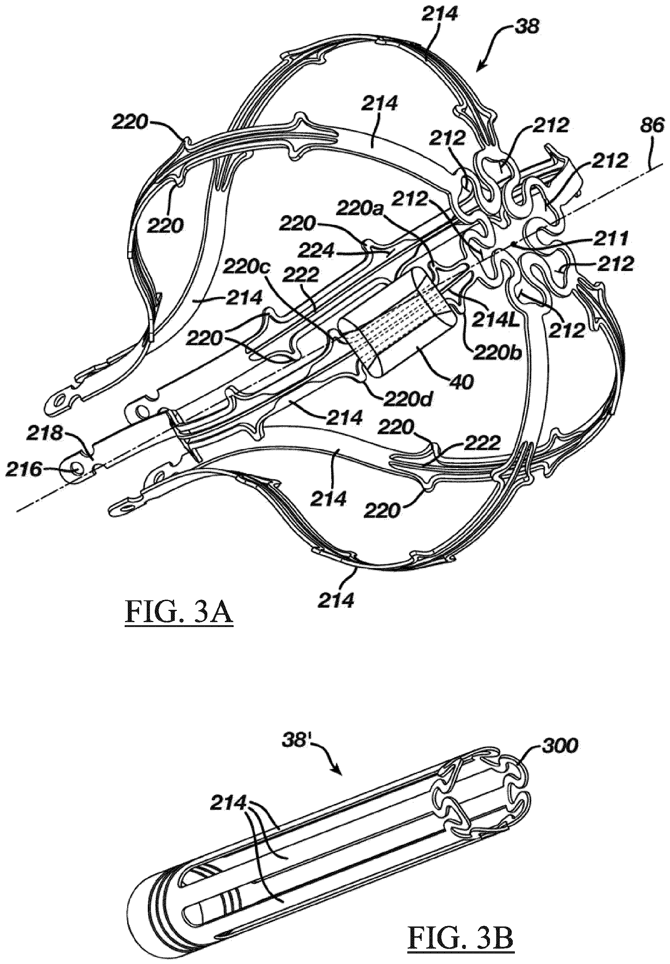

As shown in FIG. 2, basket assembly 38 includes a plurality of flexible spines 214 that are formed at the end of a tubular shaft 84 and are connected at both ends. During a medical procedure, medical professional 34 can deploy basket assembly 38 by extending tubular shaft 84 from insertion tube 30 causing basket assembly 38 to exit insertion tube 30 and transition to the expanded form. Spines 214 can have elliptical (e.g., circular), or rectangular cross-sections that may appear to be flat cross-sections, and include a flexible, resilient material (e.g., a shape-memory alloy such as nickel-titanium, also known as Nitinol) forming a strut as will be described in greater detail herein. As shown in FIGs. 2 and 3, basket assembly 38 has a proximal portion with a distal end 39. The medical probe 22 can include a spine retention hub 90 that extends longitudinally from a distal end of tubular shaft 84 towards distal end 39 of basket assembly 38. As described supra, control console 24 includes irrigation module 60 that delivers irrigation fluid to basket assembly 38 through tubular shaft 84.

Turning to FIG. 3A, the plurality of flexible linear spines 214 converge at a central spine intersection 211 that is also disposed on a longitudinal axis 86 defined by the spines 214. In some examples central spine intersection 211 can include one or more cutouts 212 that allow for bending of the spines 214 when each spine respective attachment end 216 is connected to the spine retention hub 90.

As shown herein, electrodes 40 positioned on spines 114 of basket assembly 38 can be configured to deliver ablation energy RF and/or IRE to tissue in heart 26. Additionally, or alternatively, the electrodes can also be used to determine the location of basket assembly 38 and/or to measure a physiological property such as local surface electrical potentials at respective locations on tissue in heart 26. The electrodes 40 can be biased such that a greater portion of the one or more electrodes 40 face outwardly from basket assembly 38 such that the one or more electrodes 40 deliver a greater amount of electrical energy outwardly away from the basket assembly 38 i.e., toward the heart 26 tissue than inwardly.

Examples of materials ideally suited for forming electrodes 40 include gold, platinum, and palladium and their respective alloys. These materials also have high thermal conductivity which allows the minimal heat generated on the tissue i.e., by the ablation energy delivered to the tissue to be conducted through the electrodes to the back side of the electrodes i.e., the portions of the electrodes on the inner sides of the spines, and then to the blood pool in heart 26.

Referring to FIG. 3A, basket assembly 38 of medical probe 22 is shown without the insulative sleeve 217 or associated wirings to electrodes 40 being disposed inside sleeve 217 to show the novel underlying basket structure 38. Basket 38 can include a single unitary structure that includes a plurality of spines 214 formed from a cylindrical tube stock, as illustrated in Fig. 3B, and treated to cause the spines 214 to bias radially outward. The material for the spine can be selected from a group consisting of nitinol, cobalt chromium, stainless steel, titanium, and combinations hereof.

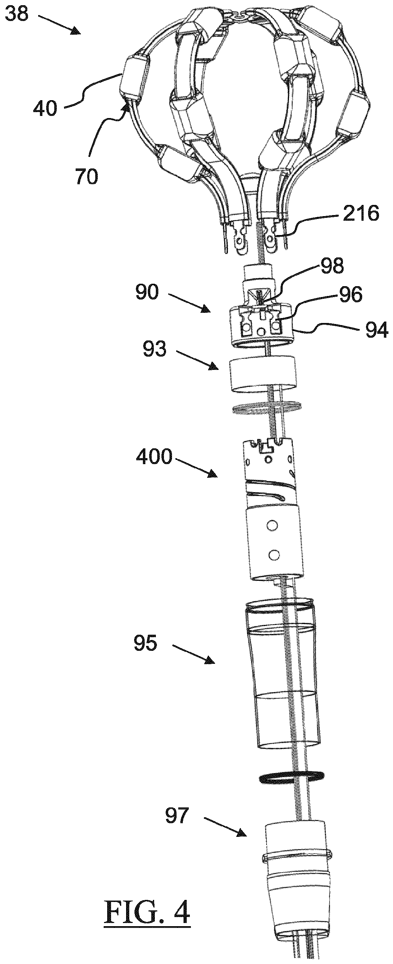

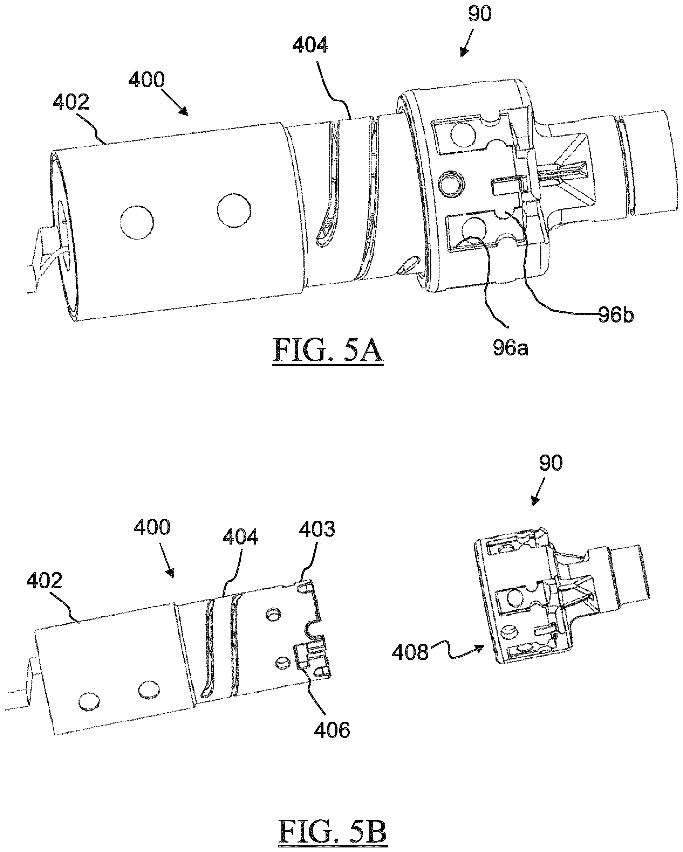

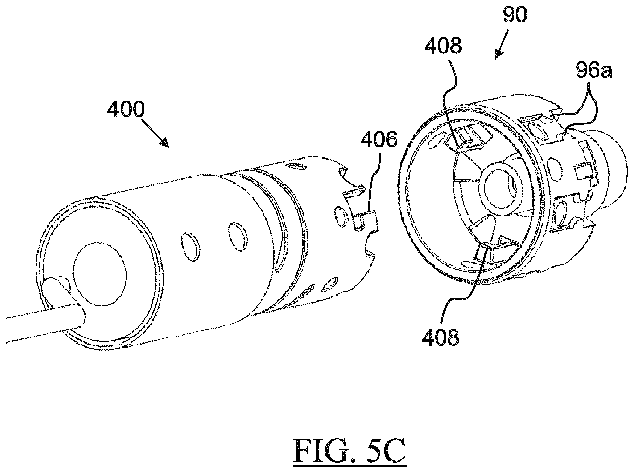

FIG. 4 illustrates an exploded view of the medical probe of FIG. 2, in accordance with an example of the disclosed technology. As illustrated in FIG. 4, the spine retention hub 90 can be inserted into the tubular shaft 84 and attached to the contact force sensor assembly 400. Spine retention hub 90 can include a cylindrical member 94 including a plurality of relief slots 96, multiple irrigation openings 98, and at least one spine retention hub electrode 99 (illustrated in FIG. 2) such as a position sensor or reference electrode, or some combination thereof. Relief slots 96 can be disposed on the outer surface of cylindrical member 94 and configured to allow a portion of each spine 214, such as each spine attachment end 216, to be fitted into a respective relief slot 96 of retention hub 90. The relief slot 96 is provided with undercuts 96a (Fig. 5A) running along the longitudinal axis to allow for insertion of spine attachment end 216 (which may have a hole as shown in Fig. 3A). Relief slot 96 may be provided with tabs 96b (Fig. 5C) that engage with complementary recess 218 on spine retention end 216 (Fig. 3A) to prevent any twisting of the spine attachment end 216 of each spine 214 with respect to the retention hub 90. This configuration of the attachment ends 216 allow the multiple spines 214 at the proximal portion of the basket 38 to act as a single structural member with the retention hub 90. The spine retention hub 90 can also act as a coupler for a contact force sensor assembly 400 as described in greater detail herein. The attachment end 216 can be a generally linear end of the spine 214. The attachment end 216 can be configured to extend outwardly from the spine retention hub 90 such that the basket assembly 38 is positioned outwardly from the spine retention hub 90 and, consequently, outwardly from the tubular shaft 84. In this way, the spine 214 can be configured to position the basket assembly 38 distally from the distal end of the tubular shaft 84 and distal from the distal end of the insertion tube 30 when the basket assembly 38 is deployed.

Electrode 40 can be located substantially in place with respect to spine 214 by way of a retention member 220 formed integrally with the spine 214. In Fig. 3A, it can be seen that each of the spines 214 can include at least one retention member 220 extending generally transverse to the spine 214. To allow for insertion of the spine 214 through a lumen 70 extending through the electrode 40, each spine 214 can be bisected with a central spine member 222 so that empty space 224 is provided to allow retention member 220 to bend inwardly toward the central spine member 222. The shape of retention member 220 can be of any shape as along as such shape serves to allow the member 220 to be compressed for insertion into lumen 70 of electrode 40 and once released to prevent movement of electrode 40 with respect to the retention member. In one example, the at least one retention member 220 is shaped in a bow-like configuration with a center of such bow extending away from a periphery of spine 214. In a preferred embodiment, the at least one retention member 220 for each electrode 40 can include two bow-shaped members 220 disposed in opposite directions and oriented transverse to a longer length 214L of each spine 214.

In the configuration shown in Fig. 3A, the at least one retention member may have first 220a, 220b and second sets 220c, 220d of retention members 220 spaced apart along the spines. The first set includes two bow-shaped members 220a, 220b disposed in opposite direction and transverse to a longer length 214L of each spine 214 and the second set includes two bow-shaped members 220c, 220d disposed in opposite direction and transverse to a longer length 214L of each spine 214 so that each electrode 40 is captured between the first and second sets of retention members 220a, 220b and 220c and 220d. Figure 3B shows the spine structure 38 as formed from a tube stock. It is also within the scope of this invention for the basket assembly 38 to be formed from a flat sheet stock, cut, and heat treated to achieve the spheroidal basket shape shown herein.

FIG. 4 illustrates an exploded view of the medical probe 22 of FIG. 2, in accordance with an example of the disclosed technology. Moving from top to bottom in FIG. 4, the assembly of the medical probe 22 will now be briefly described. As illustrated in FIG. 4, and as previously described, the spines 214 can be attached to the spine retention hub 90 to form the basket assembly 38. A spine retention sleeve 93 can be disposed over the spine retention hub to help secure the spines 214 in place. As will be described in greater detail herein, the contact force sensor assembly 400 can be coupled to the spine retention hub 90. The medical probe 22 can further include a contact force sensor assembly 400 that can be disposed in a contact force sensor assembly sleeve 95. The contact force sensor assembly sleeve 95 can be coupled to a proximal coupler 97 that can be coupled to the tubular shaft 84. When fully assembled, the basket assembly 38 can be attached to the tubular shaft 84 with the components just described to enable a medical professional 34 to insert the medical probe 22 into the heart 26 of a patient 28.

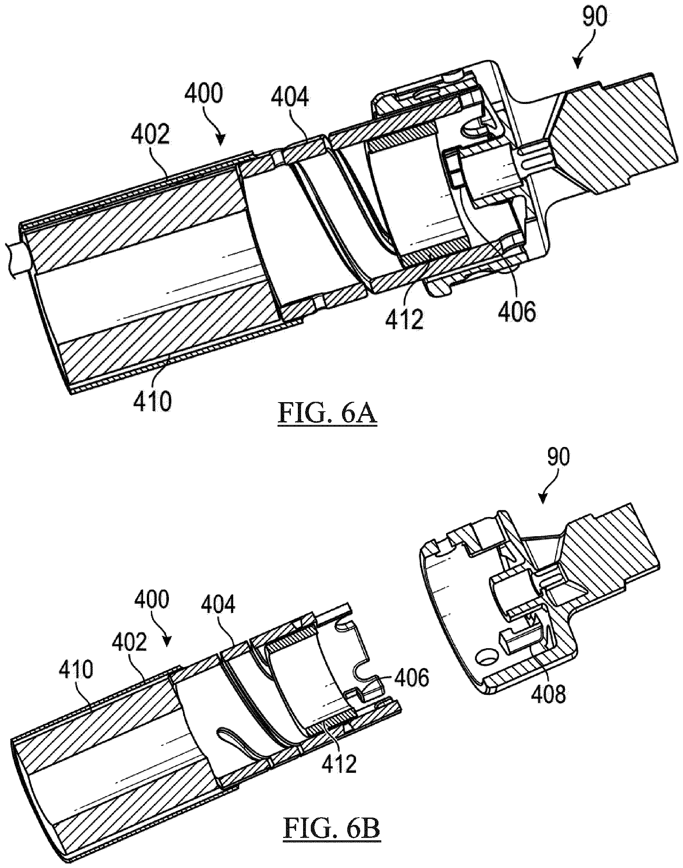

Turning now to FIGs. 5A-6B the contact force sensor assembly 400 and a spine retention hub 90 of the medical probe 22 will now be further described. Examples of contact force sensor assemblies are disclosed in U.S. Patent Nos. 8,357,152 and 10,688,278 and U.S. Patent Pub. No. 2021/0187254A1, each of which are incorporated herein by reference and attached in the appendix to priority application U.S. Provisional Patent Application No. 63/336,094. FIG. 5A illustrates a perspective view of a contact force sensor assembly 400 and a spine retention hub 90 of the medical probe 22 coupled together while FIGs. 5B and 5C illustrate exploded views of the contact force sensor assembly 400 and the spine retention hub 90. Furthermore, FIGs. 6A and 6B illustrate section views of the contact force sensor assembly 400 and the spine retention hub 90.

As illustrated, the contact force sensor assembly 400 can include a proximal end 402 and a distal end 403. The proximal end 402 can house a magnetic field generator coil 410 and the distal end 403 can house a magnetic field sensor 412. As will be appreciated, the magnetic field generator coil 410 can be configured to generate a magnetic field while the magnetic field sensor 412 can be configured to detect the presence and magnitude of the magnetic field.

The contact force sensor assembly 400 can further include a deflection portion 404 disposed between the proximal end 402 and the distal end 403. The deflection portion 404 can be configured to deflect when a force is applied to the contact force sensor assembly 400. In other words, the deflection portion 404 can be configured to permit the proximal end 402 and the distal end 403 of the contact force sensor assembly 400 to move closer to each other when a force is applied to the contact force sensor assembly 400. In one example, the deflection portion 404 can comprise a helical spring formed into a body of the contact force sensor assembly 400 as illustrated in FIGs. 5A-6B. For example, helical cuts can be made in the body of the contact force sensor assembly 400 to form a helical spring. In this way, the body of the contact force sensor assembly 400 can itself form a spring without the need for additional components. In other examples, a spring can be assembled between the proximal end 402 and the distal end 403 to form the contact force sensor assembly 400. The contact force sensor assembly 400 can be disposed inside tube 84 and proximally in relation to the basket assembly 38 and as close as possible to the basket assembly 38 so that contact with cardiac tissue by the spines 214 can be transmitted to the contact force sensor assembly 400.

As will be appreciated, when the proximal end 402 is moved closer to the distal end 403 when a force is applied to the contact force sensor assembly 400, the magnetic field sensor 412 can detect a change in the magnitude of the force of the magnetic field generated by the magnetic field generator coil 410. Because the spring constant K of the deflection portion 404 can be predetermined and the distance between the magnetic field generator coil 410 and the magnetic field sensor 412 can be detected, the force applied to the medical probe 22 can be determined (e.g., by using Hooke's law, or the equation F=d*K). Furthermore, the contact force sensor assembly 400 can receive electrical signals from, and provides electrical signals to, console 24, to process received signals and determine forces, e.g., sub-gram forces, exerted on the basket assembly 38.

As illustrated in FIGs. 5A-6B, the contact force sensor assembly 400 can include a female connector 406 while the spine retention hub 90 can include a male connector 408. As will be appreciated, however, although shown and described for convenience as the contact force sensor assembly 400 having the female connector 406 and the spine retention hub 90 having the male connector 408, the two components can be switched around without departing from the scope of this disclosure. In other words, the contact force sensor assembly 400 can include the male connector 408 while the spine retention hub 90 can include the female connector 406 depending on the particular configuration. As will be appreciated, the contact force sensor assembly 400 can include a plurality of female connectors 406 while the spine retention hub 90 can include a plurality of male connectors 408. Alternatively, the contact force sensor assembly 400 can include both a female connector 406 and a male connector 408 while the spine retention hub can include a complimentary female connector 406 and a complimentary male connector 408.

The female connector 406 and the male connector 408 can form a bayonet mount configuration in which the male connector 408 can interlock with the female connector to couple the contact force sensor assembly 400 to the spine retention hub 90. Stated otherwise, the female connector 406 can comprise a slot forming a generally "L" shape and the male connector 408 can comprise a protrusion forming a generally complimentary "L" shape. In other words, the female connector 406 can include a slot having a first slot portion extending generally longitudinally into the contact force sensor assembly 400 from a distal end 403 of the contact force sensor assembly 400 and a second slot portion extending generally transversely from an end of the first slot portion. Similarly, the male connector 408 can include a protrusion having a first protrusion portion extending generally longitudinally away from the spine retention hub 90 and a second protrusion portion extending generally transversely from an end of the first protrusion portion.

When the male connector 408 is inserted into the female connector 406, the "L" shapes of the female connector 406 and the male connector 408 can interlock, thus causing the contact force sensor assembly 400 to be coupled to the spine retention hub 90. In some examples, the male connector 408 can be caused to spring forward into the female connector 406 when the "L" portions of the female connector 406 and the male connector 408 are aligned. In this way, the male connector 408 must be pushed to remove the male connector 408 from the female connector 406 (rather than simply twisting the contact force sensor assembly 400 and/or the spring retention hub 90.

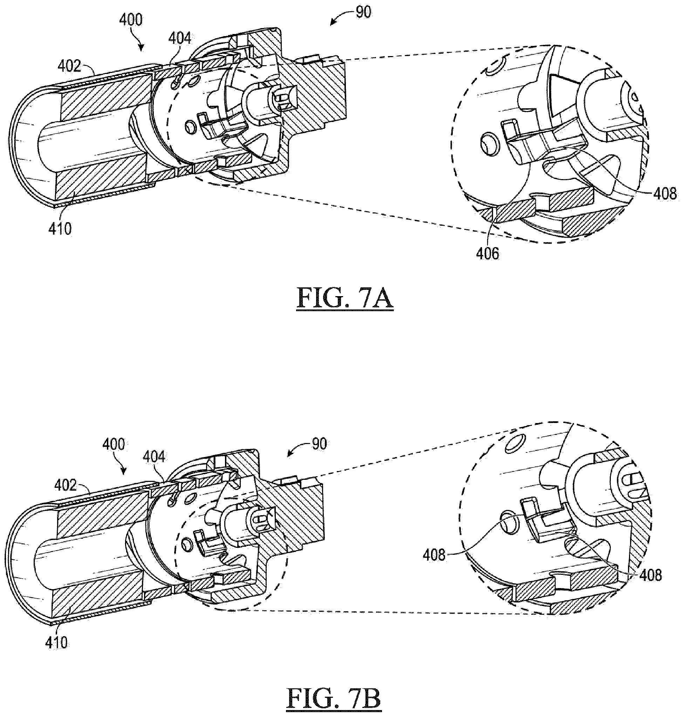

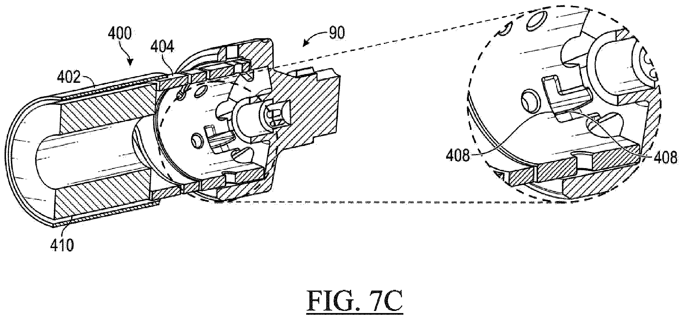

Turning now to FIGs. 7A-7C, the contact force sensor assembly 400 can be coupled to the spine retention hub 90 by aligning the female connector 406 with the male connector 408 (as illustrated in FIG. 7A), inserting the male connector 408 into the female connector 406 (as illustrated in FIG. 7B), and then twisting the contact force sensor assembly 400 and/or the spine retention hub 90 to cause the female connector 406 and the male connector 408 to interlock. In this way, the contact force sensor assembly 400 can be coupled to the spine retention hub 90 and prevent the contact force sensor assembly 400 from becoming dislodged from the spine retention hub 90 when the medical probe 22 is pulled through the flexible insertion tube 30.

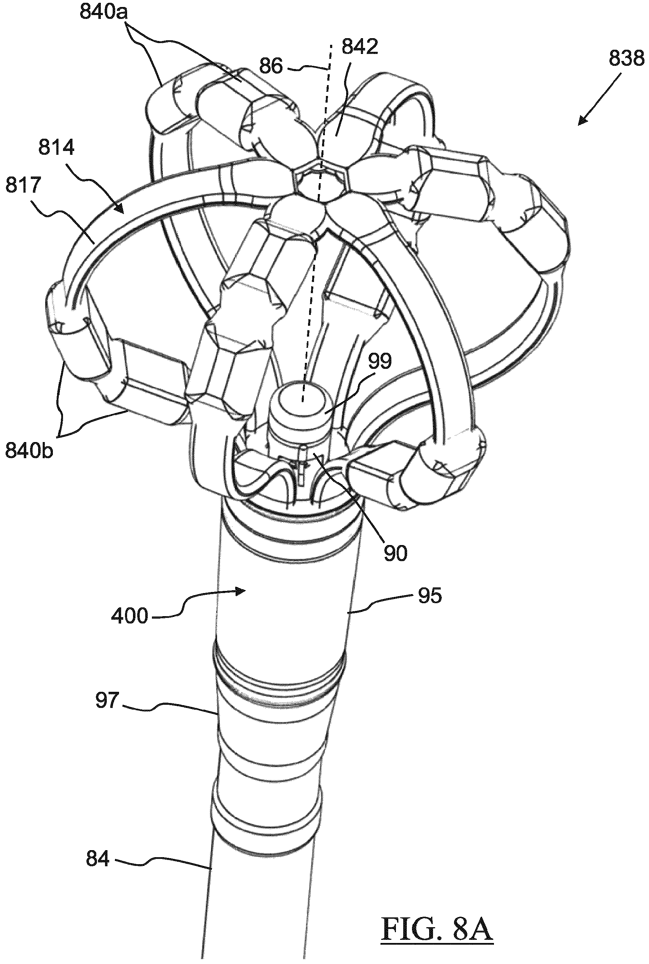

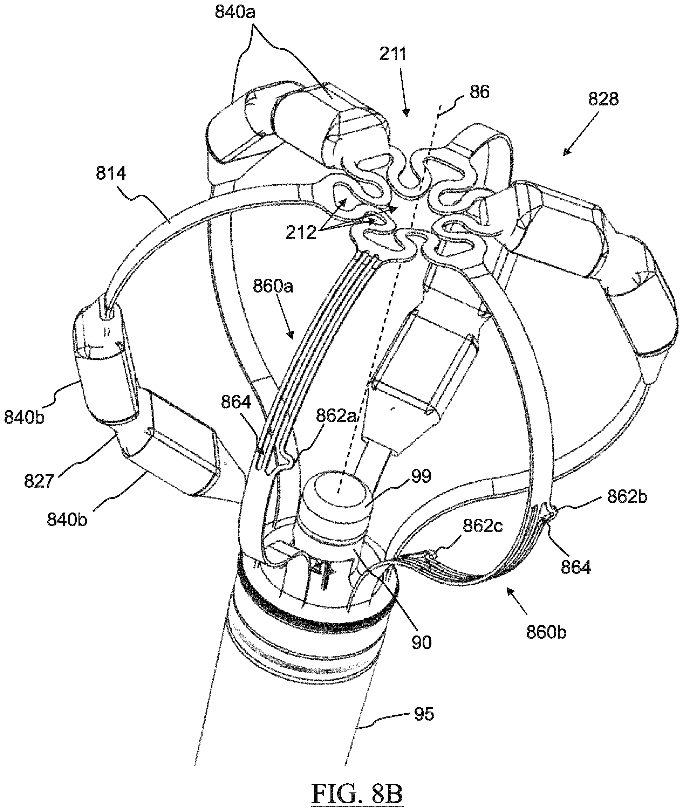

FIGs. 8A and 8B illustrate another example basket assembly 838 having a plurality of electrodes 840a, 840b disposed on spines 814 and a spine retention hub 90 having a sensor 608 mounted thereon. As shown in FIG. 8A, the electrodes 840a, 840b can be disposed in alternating groupings of distal electrodes 840a and proximal electrodes 840b on adjacent spines 814. For example, and as shown in FIGs. 8A and 8B, two electrodes 840a, 840b can be disposed on the spines 814 close to each other with no additional electrodes 840 disposed on the same spine 814. On a first spine 814, the two electrodes 840b can be disposed together near the proximal end of the spine 814 while on a second, adjacent spine 814 two electrodes 840a can be disposed together near the distal end of the adjacent spines 814. In this way, the electrodes 840a, 840b can be offset around the circumference of the basket assembly 838 such that the basket catheter 840 is better able to collapse when retracted into a sheath. When the basket assembly 838 is collapsed, the distal electrodes 840a are positioned entirely in a distal direction from the proximal electrodes 840b with a gap along the longitudinal axis 86 between the proximal electrodes 840b and the distal electrode 840a.

With the configuration of electrodes 840a, 840b disposed on the spines 814 as shown in FIGs. 8A and 8B, the medical system 20 can be configured to output bipolar high-voltage DC pulses as may be used to effect irreversible electroporation (IRE) between the two adjacent electrodes 840a, 840b on a given spine 814, electrically connect the two adjacent electrodes 840 on a given spine 814 and output bipolar high-voltage DC pulses between one or more electrodes 814 on another one of the spines 814 of the basket assembly 838, and/or output monopolar high-voltage DC pulses between one or more of the electrodes 828 and the one or more adhesive skin patches 44 disposed on the patient's 28 skin. The two electrodes 840a, 840b on a given spine 814 can include an insulative material 827 disposed between the two electrodes 840a 840b, thereby electrically isolating the two electrodes 840a 840b from each other.

As shown in FIG. 8A, the spines 814 can be covered with an insulative liner 817 that can be disposed between the electrodes 840 and the spines 814. The insulative liner 817 can electrically isolate the electrodes 840 from the spines 814 to prevent arcing or shorting to the spines 814. The insulative liner 817 can extend from the spine retention hub 90 to a distal end of the basket assembly 838. Furthermore, the insulative liner 817 can include flared ends 842 that can extend over at least a portion of the central spine intersection 211. In this way, the insulative liner 817 can have an atraumatic tip to prevent injury to tissue.

FIG. 8B is an illustration of the basket assembly 838 with the insulative liners 840, a pair of distal electrodes 840a, and a pair of proximal electrodes 840b, and other spine elements removed, for the sake of illustration, so that the frame of the basket assembly 838 is visible. As shown in FIG. 8B, the spines 814 can extend from the spine retention hub 90 and be joined together at a central spine intersection 211. The central spine intersection 211 can include one or more cutouts 212 that can allow for bending of the spines 814. The spines 814 can further include an electrode retention region 860a, 860b that is configured to prevent an electrode 840a, 840b from sliding proximally or distally along the spine 814. As shown in FIG. 8B, a first spine 814 can have distal spine retention region 860a and an adjacent spine can have a proximal spine retention region 860b. In this way, the spine retention regions 860a, 860b can be alternating between a proximal position and a distal position along the spines 814. That is, a first spine 814 can have an electrode retention region 860b disposed near a proximal end of the spine 814 and an adjacent spine 814a can have an electrode retention region 860 disposed near a distal end of the spine 814.

Each electrode retention region 860 can include one or more cutouts 864 that can permit the spine 814 to be bent or pinched inwardly. Each electrode retention region 860 can further include one or more retention members 862 that protrude outwardly and can be configured to prevent the electrode 840 from sliding proximally or distally along the spine 814. During manufacture, proximal ends of the frame of the basket assembly 838 are inserted into lumens of the electrodes 840a, 840b, and the electrodes 840a, 840b are slid distally along the spines 814 to their respective final position. The cutouts 864 permit the electrodes 840a, 840b to slide over a retention members 862a-c. Because of the one or more cutouts 864 in the spines 814, the retention members 862a-c can be configured to move inwardly when the spine 814 is pinched inwardly to permit an electrode 840a, 840b to slide over the retention member 862a-c. Once the electrode 840a, 840b is slid past the retention member 862, the retention member 862 can resiliently bend back to its previous position, thereby preventing the electrode 840a, 840b from sliding proximally or distally along the spine 814.

The proximal electrode retention region 860b includes a proximal retention member 862c and a distal retention member 862b. The proximal electrode retention region 860b need not be configured to permit the proximal electrodes 840b to pass over the distal retention member 862b. The distal electrode retention region 860a utilizes the central spine intersection 211 to prevent the distal electrodes 840a from moving distally once the distal electrodes 840a are in their respective final position.

Although the basket assembly 838 is shown as having two electrodes 840 disposed near each other on a given spine 814 and having alternating groupings of electrodes 840 on adjacent spines 814, the disclosed technology can include other configurations of electrodes 840 and spines 814 not shown. For example, the disclosed technology can include groupings of three or more electrodes 840 and/or multiple groupings of electrodes 840 disposed on spines 814. Thus, the disclosed technology is not limited to the particular configuration of electrodes 840 and spines 814 shown and described herein.

The disclosed technology described herein can be further understood according to the following clauses:
Clause 1: A medical probe, comprising: a tubular shaft including a proximal end and a distal end, the tubular shaft extending along a longitudinal axis of the medical probe; a contact force sensor assembly disposed at the distal end of the tubular shaft and configured to detect a force applied to the medical probe, the contact force sensor assembly comprising a first bayonet mount portion; a spine retention hub comprising a plurality of slots to receive respective spine members, the spine retention hub being coupled to the contact force sensor assembly, the spine retention hub having a second bayonet mount portion configured to couple the spine retention hub to the contact force sensor assembly by interlocking with the first bayonet mount portion; and an expandable basket assembly coupled to the spine retention hub, the expandable basket assembly comprising a plurality of spines disposed in respective plurality of slots of the spine retention hub and at least one electrode coupled to each of the plurality of spines, the plurality of spines extending along the longitudinal axis and configured to bow radially outward from the longitudinal axis when the expandable basket assembly is transitioned from a collapsed form to an expanded form.
Clause 2: The medical probe of clause 1, wherein the first bayonet mount comprises a slot formed into the contact force sensor, and wherein the second bayonet mount comprises a protrusion extending from the spine retention hub, the slot being configured to receive the protrusion.
Clause 3: The medical probe of clause 2, wherein the slot comprises a generally L-shaped recess, and wherein the protrusion comprises a generally L-shaped member.
Clause 4: The medical probe according to clauses 2 or 3, wherein the slot comprises a first slot portion extending generally longitudinally into the contact force sensor assembly from a distal end of the contact force sensor assembly and a second slot portion extending generally transversely from an end of the first slot portion; and wherein the protrusion comprises a first protrusion portion extending generally longitudinally away from the spine retention hub and a second protrusion portion extending generally transversely from an end of the first protrusion portion.
Clause 5: The medical probe according to any of clauses 2-4, wherein the first bayonet mount comprises at least two slots formed into the contact force sensor, and wherein the second bayonet mount comprises at least two protrusions extending from the spine retention hub, the at least two slots being configured to receive the at least two protrusions.
Clause 6: The medical probe of clause 1, wherein the first bayonet mount comprises a protrusion extending from the contact force sensor assembly, and wherein the second bayonet mount comprises a slot formed into the spine retention hub, the slot being configured to receive the protrusion.
Clause 7: The medical probe of clause 6, wherein the slot comprises a generally L-shaped recess, and wherein the protrusion comprises a generally L-shaped member.
Clause 8: The medical probe according to clauses 6 or 7, wherein the protrusion comprises a first protrusion portion extending generally longitudinally from the contact force sensor assembly and a second protrusion portion extending generally transversely from an end of the first protrusion portion; and wherein the slot comprises a first slot portion extending generally longitudinally from a proximal end of the spine retention hub and a second slot portion extending generally transversely from an end of the first slot portion.
Clause 9: The medical probe according to any of clauses 6-8, wherein the first bayonet mount comprises at least two protrusions extending from the contact force sensor, and wherein the second bayonet mount comprises at least two slots formed into the spine retention hub, the at least two slots being configured to receive the at least two protrusions.
Clause 10: The medical probe according to any of clauses 1-9, wherein the contact force sensor assembly comprises: a body having a generally cylindrical shape; a coil configured to generate a magnetic field; a sensor configured to detect the magnetic field generated by the coil; and a deflection portion configured to permit the body to deflect when a force is applied to the contact force sensor assembly.
Clause 11: The medical probe of clause 10, wherein the deflection portion comprises a helical spring.
Clause 12: The medical probe of clause 11, wherein the helical spring is formed into the body of the contact force sensor by forming a helical cut into the body of the contact force sensor.
Clause 13: The medical probe according to any of clauses 10-12, wherein the coil is disposed at a proximal end of the contact force sensor assembly and the sensor is disposed at a distal end of the contact force sensor assembly.
Clause 14: The medical probe according to any of clauses 1-13, wherein the spine retention hub comprises a spray port configured to direct a fluid toward the at least one electrode.
Clause 15: The medical probe according to any of clauses 1-14, wherein the plurality of spines each include at least one retention member extending generally transverse to the spine.
Clause 16: The medical probe of clause 15, wherein the at least one electrode comprises a body defining a hollow portion extending through the body of the electrode, the body configured to receive each of the plurality of spines, the electrode retained by the at least one retention member.
Clause 17: The medical probe according to clauses 15 or 16, in which the at least one retention member comprises a bow-shaped member.
Clause 18: The medical probe according to any of clauses 15-17, in which the at least one retention member comprises two bow-shaped members disposed in opposite direction and transverse to a longer length of each spine.
Clause 19: The medical probe according to any of clauses 15-18, wherein the at least one electrode comprises a first electrode and a second electrode; and wherein the at least one retention member comprises first and second sets of retention members spaced apart along each of the plurality of spines, the first set includes two bow-shaped members disposed in opposite direction and transverse to a longer length of each spine and the second set includes two bow-shaped members disposed in opposite direction and transverse to a longer length of each spine so that the first electrode is captured between the first set of retention members and the second electrode is captured between the second set of retention members.
Clause 20: The medical probe according to any of clauses 1-19, further comprising an electrically insulative jacket disposed between each of the plurality of spines and the at least one electrode, thereby electrically isolating the at least one electrode from each of the plurality of spines.
Clause 21: The medical probe according to clause 20 , further comprising a wire disposed inside the insulative jacket.
Clause 22: The medical probe according to clause 21, wherein the wire is electrically connected to the at least one electrode.
Clause 23: The medical probe according to any of clauses 1-22, wherein each of the plurality of spines comprise a material selected from a group consisting of nitinol, cobalt chromium, stainless steel, titanium, and combinations hereof.
Clause 24: The medical probe according to any of clauses 1-23, wherein at least one electrode comprises of a material selected from stainless steel, cobalt chromium, gold, platinum, palladium, and alloys hereof.
Clause 25: The medical probe according to any of clauses 1-24, wherein the at least one electrode is configured to deliver electrical pulses for irreversible electroporation, the pulses including a peak voltage of at least 900 volts (V).
Clause 26: The medical probe according to any of clauses 1-25, wherein each of the plurality of spines is configured to form an approximately spherically-shaped basket assembly when in the expanded form.
Clause 27: The medical probe according to any of clauses 1-26, wherein each of the plurality of spines is configured form an approximately oblate-spheroid basket assembly when in the expanded form.
Clause 28: A medical probe, comprising: a tubular shaft extending along a longitudinal axis of the medical probe; a contact force sensor assembly disposed at the distal end of the tubular shaft and configured to detect a force applied to the medical probe, the contact force sensor assembly comprising a first bayonet mount portion; a plurality of spines configured to bow radially outward from the longitudinal axis, each spine of the plurality of spines comprising a retention member; a plurality of electrodes, each electrode of the plurality of electrodes attached to a spine of the plurality of spines and prevented from sliding proximally or distally along the spine by the retention member, the plurality of electrodes being disposed on the plurality of spines in groupings, the groupings being disposed in alternating proximal and distal positions along adjacent spines; and a spine retention hub comprising a plurality of slots to receive respective spines of the plurality of spines, the spine retention hub having a second bayonet mount portion configured to couple the spine retention hub to the contact force sensor assembly by interlocking with the first bayonet mount portion.

The embodiments described above are cited by way of example, and the present invention is not limited by what has been particularly shown and described hereinabove. Rather, the scope of the invention includes both combinations and sub combinations of the various features described and illustrated hereinabove, as well as variations and modifications thereof which would occur to persons skilled in the art upon reading the foregoing description and which are not disclosed in the prior art.

## Claims

1. A medical probe, comprising:
a tubular shaft including a proximal end and a distal end, the tubular shaft extending along a longitudinal axis of the medical probe;
a contact force sensor assembly disposed at the distal end of the tubular shaft and configured to detect a force applied to the medical probe, the contact force sensor assembly comprising a first bayonet mount portion;
a spine retention hub comprising a plurality of slots to receive respective spine members, the spine retention hub being coupled to the contact force sensor assembly, the spine retention hub having a second bayonet mount portion configured to couple the spine retention hub to the contact force sensor assembly by interlocking with the first bayonet mount portion; and
an expandable basket assembly coupled to the spine retention hub, the expandable basket assembly comprising a plurality of spines disposed in a respective plurality of slots of the spine retention hub and at least one electrode coupled to each of the plurality of spines, the plurality of spines extending along the longitudinal axis and configured to bow radially outward from the longitudinal axis when the expandable basket assembly is transitioned from a collapsed form to an expanded form.

2. The medical probe of claim 1, wherein the first bayonet mount comprises a slot formed into the contact force sensor, and
wherein the second bayonet mount comprises a protrusion extending from the spine retention hub, the slot being configured to receive the protrusion.

3. The medical probe of claim 2, wherein the slot comprises (i) a generally L-shaped recess, and wherein the protrusion comprises a generally L-shaped member and/or (ii) a first slot portion extending generally longitudinally into the contact force sensor assembly from a distal end of the contact force sensor assembly and a second slot portion extending generally transversely from an end of the first slot portion; and
wherein the protrusion comprises a first protrusion portion extending generally longitudinally away from the spine retention hub and a second protrusion portion extending generally transversely from an end of the first protrusion portion.

4. The medical probe of claim 2 or claim 3, wherein the first bayonet mount comprises at least two slots formed into the contact force sensor, and
wherein the second bayonet mount comprises at least two protrusions extending from the spine retention hub, the at least two slots being configured to receive the at least two protrusions.

5. The medical probe of claim 1, wherein the first bayonet mount comprises a protrusion extending from the contact force sensor assembly, and
wherein the second bayonet mount comprises a slot formed into the spine retention hub, the slot being configured to receive the protrusion, optionally wherein the slot comprises a generally L-shaped recess, and
wherein the protrusion comprises a generally L-shaped member.

6. The medical probe according to claim 5, wherein the protrusion comprises a first protrusion portion extending generally longitudinally from the contact force sensor assembly and a second protrusion portion extending generally transversely from an end of the first protrusion portion; and
wherein the slot comprises a first slot portion extending generally longitudinally from a proximal end of the spine retention hub and a second slot portion extending generally transversely from an end of the first slot portion.

7. The medical probe of any preceding claim, wherein the contact force sensor assembly comprises:
a body having a generally cylindrical shape;
a coil configured to generate a magnetic field;
a sensor configured to detect the magnetic field generated by the coil; and
a deflection portion configured to permit the body to deflect when a force is applied to the contact force sensor assembly, optionally wherein the deflection portion comprises a helical spring, further optionally wherein the helical spring is formed into the body of the contact force sensor by forming a helical cut into the body of the contact force sensor.

8. The medical probe of any preceding claim, wherein the spine retention hub comprises a spray port configured to direct a fluid toward the at least one electrode.

9. The medical probe of claim 1, wherein the plurality of spines each include at least one retention member extending generally transverse to the spine, the plurality of spines comprising a first spine including a single cross-section extending from a proximal portion to approximately a midpoint of the first spine and thereafter dividing into at least two discrete cross sections to the distal portion of the first spine, and
a second spine including at least two discrete cross sections extending from a proximal portion to approximately a midpoint of the second spine and thereafter combining into a single cross section extending to the distal portion of the second spine.

10. The medical probe of claim 9, wherein (i) the at least one electrode comprises a body defining a hollow portion extending through the body of the electrode, the body configured to receive each of the plurality of spines, the electrode retained by the at least one retention member, or (ii) the at least one retention member comprises a bow-shaped member.

11. A medical device comprising:
a contact force sensor assembly configured to detect a force applied to an end effector of the medical device, the contact force sensor assembly comprising a first bayonet mount portion;
an end effector retention hub configured to receive at least a portion of the end effector to secure the end effector to the end effector retention hub, the end effector retention hub having a second bayonet mount portion configured to couple the end effector retention hub to the contact force sensor assembly by interlocking with the first bayonet mount portion.

12. The medical device of claim 11, wherein the contact force sensor assembly comprises:
a body having a generally cylindrical shape;
a coil configured to generate a magnetic field;
a sensor configured to detect the magnetic field generated by the coil; and
a helical spring formed into the body and configured to permit the body to deflect when a force is applied to the end effector.

13. The medical device of claim 11, wherein the end effector retention hub comprises a spray port configured to direct a fluid toward an electrode of the end effector.

14. The medical device of claim 11, wherein the first bayonet mount comprises a slot formed into the contact force sensor, and
wherein the second bayonet mount comprises a protrusion extending from the end effector retention hub, the slot being configured to receive the protrusion.

15. A medical probe, comprising:
a tubular shaft extending along a longitudinal axis of the medical probe;
a contact force sensor assembly disposed at the distal end of the tubular shaft and configured to detect a force applied to the medical probe, the contact force sensor assembly comprising a first bayonet mount portion;
a plurality of spines configured to bow radially outward from the longitudinal axis, each spine of the plurality of spines comprising a retention member;
a plurality of electrodes, each electrode of the plurality of electrodes attached to a spine of the plurality of spines and prevented from sliding proximally or distally along the spine by the retention member, the plurality of electrodes being disposed on the plurality of spines in groupings, the groupings being disposed in alternating proximal and distal positions along adjacent spines; and
a spine retention hub comprising a plurality of slots to receive respective spines of the plurality of spines, the spine retention hub having a second bayonet mount portion configured to couple the spine retention hub to the contact force sensor assembly by interlocking with the first bayonet mount portion.
